# EUROPEAN PATENT APPLICATION

(11) **EP 4 406 527 A1**
(43) Date of publication of application: **31.07.2024**
(21) Application number: 22872882.0
(22) Date of filing: 20.09.2022
(51) Int. Cl.: A61K 8/19, A61Q 5/00, A61Q 19/00, A61M 11/00, A45D 44/00, A61H 33/12

(54) **TOPICAL AGENT FOR SKIN AND HAIR, COSMETIC OPERATION METHOD, AND FINE WATER PARTICLE SUPPLY DEVICE**

(30) Priority: 21.09.2021 JP 2021152888; 21.09.2021 JP 2021152889; 07.10.2021 JP 2021165726
(71) Applicant: AISIN CORPORATION, Kariya-shi, Aichi 448-8650 (JP)
(72) Inventor: HIRANO, Akiyoshi, Kariya-shi, Aichi 448-8650 (JP); INOUE, Shinsuke, Kariya-shi, Aichi 448-8650 (JP); SHIGEMORI, Yasushi, Kariya-shi, Aichi 448-8650 (JP); TABATA, Yuki, Kariya-shi, Aichi 448-8650 (JP)
(74) Representative: Cabinet Beau de Loménie
(86) International application number: PCT/JP2022/034955
(87) International publication number: WO 2023/048130

(57) **Abstract**

A cosmetic treatment method includes: applying, to skin or hair, an external preparation including a predetermined active ingredient and a base, and at least one of the active ingredient and the base contains fine water that is uncharged and has a particle size of less than or equal to 50 nanometers. Therefore, the applied fine water enters the skin or hair to contribute to maintenance of moisturization, and at the same time, forms a route through which the active ingredient permeates, thereby promoting the permeation of the active ingredient; therefore, the permeability of the active ingredient can be more appropriately improved.

## Description

### TECHNICAL FIELD

The present disclosure relates to an external preparation for skin or hair, a cosmetic treatment method, and a fine water supply device.

### BACKGROUND ART

Conventionally, there have been proposed a product, a method, and a device that promote permeability, to skin, of an external preparation such as a cosmetic liquid applied to the skin. For example, Patent Literature 1 describes a method and a device to increase a permeation amount of an external preparation by generating charged fine particles by using discharge in the atmosphere and releasing the charged fine particles to the external preparation that is applied to skin. Patent Literature 2 describes a method and a device to promote permeation of a cosmetic component into skin by applying the cosmetic component to the skin and then irradiating the skin with positive ions and negative ions that are bonded to water molecules by discharge in the atmosphere.

### CITATIONS LIST

### PATENT LITERATURE

Patent Literature 1: JP 2016 -59703 A
Patent Literature 2: WO 2015/156017 A

### SUMMARY OF INVENTION

### TECHNICAL PROBLEMS

However, when an external preparation or water particles are charged by discharge or the like as described above, the external preparation or the water particles are electrically sucked by a surface of a horny layer of the skin, and the permeability into the horny layer may be rather reduced. Furthermore, in the case where ions are further irradiated after the cosmetic component is applied to the skin, it takes time to perform a treatment.

The present disclosure is to more appropriately improve the permeability of an active ingredient when an external preparation is applied to skin or hair.

### SOLUTIONS TO PROBLEMS

The present disclosure has adopted the following means to achieve the above product, method, and device.

An external preparation of the present disclosure is an external preparation for skin or hair and includes: a predetermined active ingredient; and a base. At least one of the active ingredient and the base contains fine water that is uncharged and has a particle size of less than or equal to 50 nanometers.

The external preparation of the present disclosure contains, in at least one of the active ingredient and the base, fine water that is uncharged and has a particle size of less than or equal to 50 nanometers. When the external preparation is applied to skin or hair, the fine water enters the skin or hair to contribute to maintenance of moisturization, and at the same time, forms a route through which the active ingredient permeates, thereby promoting the permeation of the active ingredient; therefore, the permeability of the active ingredient can be more appropriately improved.

A first cosmetic treatment method of the present disclosure includes: applying, to skin or hair, an external preparation including a predetermined active ingredient and a base, and at least one of the active ingredient and the base contains fine water that is uncharged and has a particle size of less than or equal to 50 nanometers. Therefore, it is possible to more appropriately improve the permeability of the active ingredient when the external preparation is applied to the skin or hair. The cosmetic treatment method may include supplying fine water to the external preparation until the step of applying is started. In this way, the external preparation can be applied in a state where the external preparation is prevented from being dried and sufficiently contains fine water, so that the permeation effect can be reliably improved.

A second cosmetic treatment method of the present disclosure includes: disposing, in a tank, a base containing a predetermined active ingredient or an external preparation having the base, as a target agent; increasing a concentration of the fine water in the tank, by repeating a moisture absorption state to adsorb moisture in the air to the conductive polymer film in which air outside the tank is supplied to a conductive polymer film at a lowered temperature while an inside of the tank is sealed, and a moisture release state to cause the moisture adsorbed to the conductive polymer film to be released into the tank as fine water having a particle size of less than or equal to 50 nanometers in which the air in the tank is circulated to the conductive polymer film at a raised temperature; supplying the fine water to the target agent for a predetermined time; and taking out the target agent from the tank and applying the target agent to skin or hair as an external preparation.

In the second cosmetic treatment method of the present disclosure, the fine water is supplied to the target agent for a predetermined time in a state where the concentration of the fine water in the tank is increased, and the target agent is taken out from the tank and applied to skin or hair as an external preparation. Therefore, the target agent is applied after the fine water is made to be sufficiently contained in the target agent, so that the permeability of the active ingredient can be further improved while enhancing a moisturizing effect.

A fine water supply device of the present disclosure includes: a fine water generator that changes to a moisture absorption state in which moisture in air is made to be adsorbed to a conductive polymer film due to a decrease in temperature and to a moisture release state in which the moisture adsorbed to the conductive polymer film is made to be released into a tank as fine water having a particle size of less than or equal to 50 nanometers due to an increase in temperature; and a control unit that controls the fine water generator such that, in a state where a base containing a predetermined active ingredient or an external preparation having the base is disposed in the tank as a target agent, the fine water is supplied to the target agent.

In the fine water supply device of the present disclosure, the fine water generator is controlled such that the fine water is supplied to the target agent in a state where the base or the external preparation is disposed in the tank as the target agent, so that the external preparation containing the fine water can be relatively easily produced. When the external preparation is applied to skin or hair, the permeability of the active ingredient can be more appropriately improved.

The fine water supply device of the present disclosure may include a stirring unit that stirs the target agent in the tank, and the control unit may control the stirring unit such that the stirring unit stirs the target agent in at least one of the moisture absorption state and the moisture release state. This configuration makes it possible to cause the fine water to be uniformly contained in the base; therefore, the permeability of the active ingredient can be further improved while enhancing the moisturizing effect.

The fine water supply device of the present disclosure may include a switching part that selects any one of a sealed state in which communication between the fine water generator and the tank is blocked and the air outside the tank is supplied to the fine water generator, and a circulation state in which communication is established between the fine water generator and the tank and air inside the tank is supplied to the fine water generator. The control unit may bring, in the sealed state of the switching part, the fine water generator into the moisture absorption state and may bring, in the circulation state of the switching part, the fine water generator into the moisture release state such that the concentration of the fine water in the tank is increased. This configuration makes it possible to bring the inside of the tank in a high concentration environment of fine water and to cause the fine water to be sufficiently contained in the base.

The first cosmetic treatment method of the present disclosure may include: adsorbing moisture to a conductive polymer film having a nanochannel; increasing a temperature of the conductive polymer film to release the moisture adsorbed to the conductive polymer film; and causing the fine water to be contained in at least one of the active ingredient and the base. This configuration makes it possible to cause fine water having a smaller particle size to be contained, so that the permeability of the active ingredient can be further improved.

### BRIEF DESCRIPTION OF DRAWINGS

FIG. 1 is a configuration diagram schematically illustrating a configuration of a fine water supply device 10.
FIG. 2A is a configuration diagram schematically illustrating a configuration of a fine water generation cartridge 30, and FIG. 2B is a configuration diagram schematically illustrating a configuration of a fine water release element 34.
FIG. 3 is a process chart illustrating an example of an external preparation producing method.
FIG. 4 is a flowchart illustrating an example of a fine water supply process.
FIG. 5 is a configuration diagram schematically illustrating a configuration of a fine water permeation chamber 15.
FIG. 6 is a process chart illustrating an example of an external preparation application method.
FIG. 7 is an explanatory view illustrating an image in which fine water permeates into skin.
FIG. 8 is a configuration diagram schematically illustrating a configuration of a fine water supply device 100 of a second embodiment.
FIG. 9 is a flowchart illustrating an example of a fine water supply process of the second embodiment.

### DESCRIPTION OF EMBODIMENTS

### First embodiment

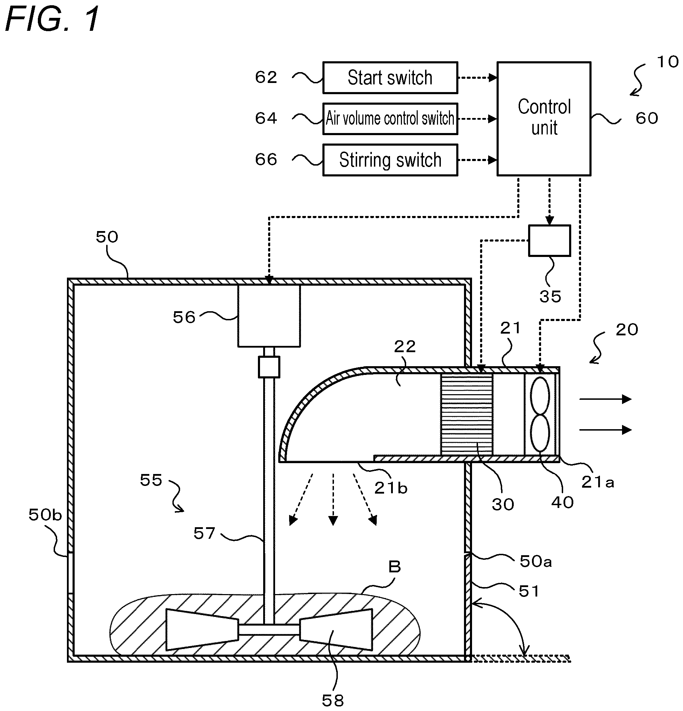

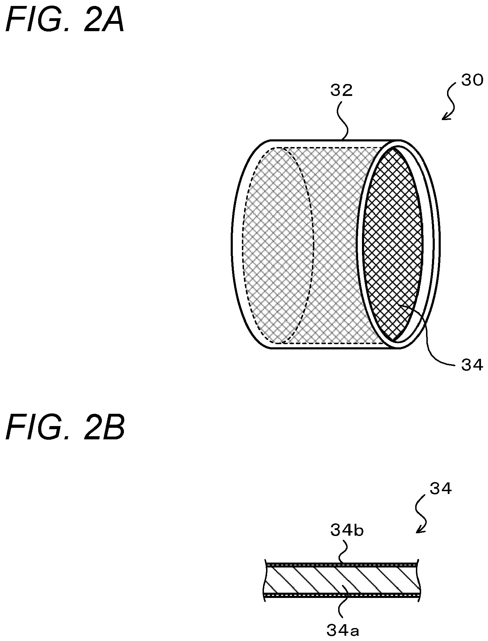

Next, a first embodiment of the present disclosure will be described with reference to the drawings. FIG. 1 is a configuration diagram schematically illustrating a configuration of a fine water supply device 10 of a first embodiment, FIG. 2A is a configuration diagram schematically illustrating a configuration of a fine water generation cartridge 30, and FIG. 2B is a configuration diagram schematically illustrating a configuration of a fine water release element 34. The fine water supply device 10 includes: a fine water supply unit 20 that supplies fine water; a supply tank 50 that contains a supply target of the fine water; and a control unit 60 that controls the entire device.

Here, the supply target of the fine water is an external preparation P to be applied to skin, hair, or the like, or a base B serving as a base component of the external preparation P. Examples of the external preparation P to be applied to skin include cosmetics such as lotions, serums, milky lotions, and creams, and external preparations such as ointments, gels, and creams. Furthermore, examples of the external preparation P to be applied to hair include hair care preparations such as coloring agents, treatment agents, and perming agents. Furthermore, the base B is mixed with a cosmetic component, a medicinal component, or the like as a predetermined active ingredient. Examples of the base B and the active ingredient include, without being limited thereto: moisturizing components, herbal extracts, enzymes such as tyrosinase, superoxide dismutase, and lipase; vitamins and derivatives thereof such as retinol, ascorbic acid, tocopherol, pyridoxal, and riboflavin; organic dyes such as β-carotene and chlorophyll; moisture components such as glycerin, sorbitol, urea, lactic acid, propylene glycol, polyethylene glycol, and copolymers, and glucose derivatives; emollient components such as paraffin, stearyl alcohol, cetyl alcohol, squalene, silicone oil, and stearyl; treatment components; dandruff inhibition components; pilatory and hair growth components; and the like.

As illustrated in FIG. 1, the fine water supply unit 20 includes a duct 21 in which an air passage 22 is formed, the fine water generation cartridge 30, an energization circuit 35, and a fan 40. The fine water supply unit 20 is attached to the supply tank 50 such that the duct 21 penetrates through a side wall of the supply tank 50.

The duct 21 is a tubular member whose both ends are opened, and has an opening 21a opened laterally (horizontally) outside the supply tank 50 and an opening 21b opened downward (vertically) inside the supply tank 50. The duct 21 and the air passage 22 extend linearly in the horizontal direction from the opening 21a to the inside of the supply tank 50, and then extend so as to curve downward toward the opening 21b. The fine water generation cartridge 30 and the fan 40 are disposed in a straight portion of the air passage 22 in the order of the fan 40 and the fine water generation cartridge 30 from the opening 21a side.

As illustrated in FIG. 2A, the fine water generation cartridge 30 includes: a case 32 in a cylindrical shape having such an outer diameter that the case can be disposed in the air passage 22; and a fine water generation element 34 provided in the case 32. As shown in FIG. 2B, the fine water generation element 34 includes a base material 34a and a conductive polymer film 34b formed on the surface of the base material 34a.

The base material 34a is formed of a conductive material such as a metal material including a stainless steel-based metal or a copper-based metal, a carbon material, or a conductive ceramic material. In the present embodiment, a metal foil of stainless steel to which aluminum is added is used. The fine water generation element 34 is formed in a corrugated plate shape, a honeycomb shape, a spiral shape, or the like so that air can flow through and a surface area of the base material 34a (conductive polymer film 34b) is as large as possible. An energization circuit 35 including a power supply and a switch is connected to the base material 34a. When the switch is turned on by the control unit 60, the energization circuit 35 enters an energization state for energizing the base material 34a, and when the switch is turned off by the control unit 60, the energization circuit 35 enters a non-energization state for cutting off energization to the base material 34a.

The conductive polymer film 34b is formed of a polymer compound having conductivity such as a thiophene-based conductive polymer. In the present embodiment, the conductive polymer 34b is formed of PEDOT/PSS (poly (3,4-ethylenedioxythiophene)/poly (styrenesulfonic acid)) among thiophene-based conductive polymers. PEDOT/PSS has a structure in which PEDOT is dispersed in PSS having a sulfonic acid group, which is a hydrogen bondable acidic functional group. In addition, nanochannels which are channels having a nanometer size of about 2 nanometers (nm) are formed at boundary portions between PEDOT and PSS. Because a large amount of a sulfonic acid group is present in the nanochannels, moisture present on the surface of conductive polymer film 34b moves inside along the sulfonic acid group in the nanochannels due to a concentration difference between the surface and the inside when the amount of moisture on the surface is large and the amount of moisture in the inside is small. Consequently, the conductive polymer film 34b adsorbs moisture. In addition, when the amount of moisture on the surface is small and the amount of moisture inside is large in a state where the moisture is adsorbed inside, the moisture moves to the surface along the sulfonic acid group in the nanochannels due to the concentration difference between the surface and the inside. Consequently, moisture is released as fine water from the conductive polymer film 34b. Furthermore, in a state where the temperature of the conductive polymer film 34b has increased, rapid release of moisture (fine water) is promoted as compared with a case where the moisture moves only by the concentration difference, and in a state where the temperature of conductive polymer film 34b has decreased, rapid adsorption of moisture is promoted as compared with a case where the moisture moves only by the concentration difference. As described above, the fine water generation cartridge 30 (fine water generation element 34) changes to a moisture absorption state where moisture in the air is adsorbed to the conductive polymer film 34b due to a decrease in temperature, and changes to a moisture release state where the adsorbed moisture is released from the conductive polymer film 34b due to an increase in temperature. Note that a thickness of the conductive polymer film 34b can be appropriately determined in accordance with a required adsorption amount (release amount) of the fine water. For example, when conductive polymer film 34b is formed to have a thickness of, for example, 1 to 30 micrometers, moisture sufficient to release fine water can be adsorbed to the conductive polymer film 34b in about several seconds to several tens of seconds.

In addition, the fine water generation cartridge 30 releases, from the conductive polymer film 34b of the fine water generation element 34, uncharged fine water having a water particle size of 50 nanometers or less, for example, a particle size of about 1 to 2 nanometers. The reason that the particles have such a particle size is considered as follows. Since the size of the nanochannels is 2 nanometers or less, the increase in the temperature of the conductive polymer film increases the mobility and a pressure of water in the nanochannels, so that the moisture jumps out from the nanochannels. In addition, even when the water particles aggregate after jumping out, the particle size of the aggregation is distributed in the range of 50 nanometers or less. A detailed description about such generation of fine water in the fine water generation cartridge 30 (conductive polymer film 34b) is described in WO 2020/054100 A, JP 2019-018195 A, and the like of the applicant of the present application; therefore, a further detailed description is omitted.

The fan 40 rotates in a first rotation direction to blow air from the opening 21a outside the supply tank 50 toward the opening 21b inside the supply tank 50. Therefore, the air sucked into the air passage 22 can be sent into the supply tank 50 through the fine water generation cartridge 30. In addition, being rotationally driven in a second rotation direction opposite to the first rotation direction, the fan 40 blows air from the opening 21b in the supply tank 50 toward the opening 21a outside the supply tank 50. The fan 40 is rotationally driven by a motor (not illustrated), and is controlled by the control unit 60 by pulse width modulation (PWM) control, voltage control, or the like. Note that the fan 40 may be a propeller fan, a sirocco fan, or the like.

The supply tank 50 is a case having a rectangular parallelepiped shape or a tubular shape, the fine water supply unit 20 is attached to the side wall as described above, and there are formed, in the side walls, an opening 50a for putting in and taking out the base B as the supply target or the like and an exhaust port 50b for exhausting the air in the supply tank 50 to the outside. The opening 50a can be manually opened and closed with an opening and closing plate 51. In addition, the supply tank 50 is provided with a stirring device 55 for stirring the base B and the like as the supply target.

The stirring device 55 includes: a motor 56 fixed to an upper wall of the supply tank 50; a shaft 57 connected to a rotation shaft of the motor 56 and extending downward; and a plurality of stirring blades 58 radially extending in the horizontal direction from a lower end of the shaft 57. The stirring device 55 transmits a rotational driving force of the motor 56 to the stirring blades 58 via the shaft 57, and rotates the stirring blades 58 to stir the base B and the like.

The control unit 60 is configured as a microprocessor mainly configured with a CPU, and includes a ROM, a RAM, and input and output ports in addition to the CPU. To the control unit 60 there are input, via the input port: an operation signal from a start switch 62 to start an operation of the fine water supply device 10 (fine water supply unit 20); an operation signal from an air volume control switch 64 to control an air volume of the fan 40; an operation signal from a stirring switch 66 to set the presence or absence of the stirring operation by the stirring device 55; and other signals. Furthermore, from the control unit 60 there are output, via the output port: a drive signal to the motor for rotationally driving the fan 40; a drive signal to the switch of the energization circuit 35; a drive signal to the motor 56 of the stirring device 55; and other signals.

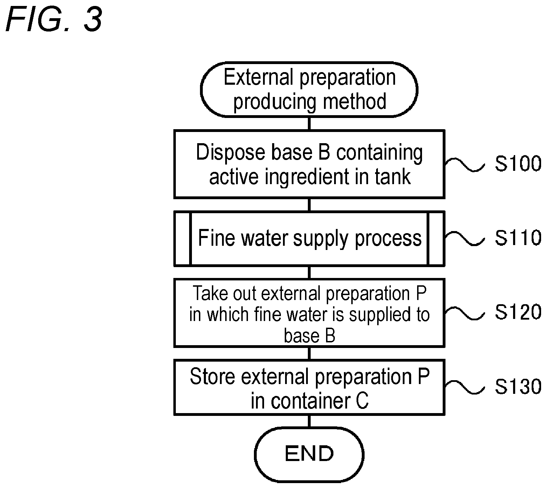

Next, a description will be given on a method for producing the external preparation P by using the fine water supply device 10 configured as described above and an application method for applying the produced external preparation P to skin or hair. FIG. 3 is a process chart illustrating an example of an external preparation producing method. An operator such as a manufacturer of the external preparation P first disposes the base B containing the above-mentioned active ingredient in the supply tank 50 (step S100). Next, the operator operates the start switch 62 to perform the fine water supply process by the fine water supply unit 20 (step S110). Note that it is assumed here that the stirring switch 66 has been turned on and "stirring operation is performed" has thus been set.

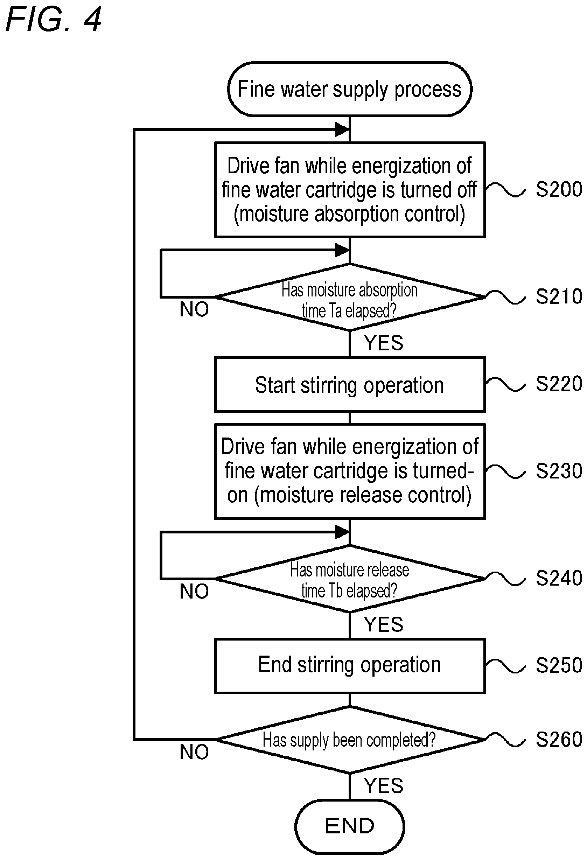

FIG. 4 is a flowchart illustrating an example of the fine water supply process. In the fine water supply process, the control unit 60 performs moisture absorption control in which the fan 40 is driven while energization to the fine water generation cartridge 30 is turned off so that moisture adsorbs to the conductive polymer film 34b (S200), and the controller waits for a predetermined moisture absorption time Ta to elapse (S210). In the moisture absorption control, the control unit 60 drives the fan 40 to rotate in the second rotation direction. Therefore, the air sucked into the air passage 22 from the opening 21b in the supply tank 50 is released from the opening 21a (see the solid arrows in FIG. 1). In addition, since control unit 60 turns off the energization circuit 35, the energization circuit 35 enters the non-energization state in which energization to the base material 34a is cut off, and the temperature of the conductive polymer film 34b decreases, thereby promoting adsorption of moisture.

If the control unit 60 determines in S210 that the moisture absorption time Ta has elapsed, the control unit 60 activates the stirring device 55 to start the stirring operation of the base B (S220), and the control unit 60 drive the fan 40 in the first rotation direction to perform moisture release control while the energization to the fine water generation cartridge 30 is turned on (S230). In the moisture release control, the control unit 60 rotationally drives the fan 40 in the first rotation direction. Therefore, the air sucked into the air passage 22 from the opening 21a outside the supply tank 50 is released into the supply tank 50 from the opening 21b (see the dotted arrows in FIG. 1). Furthermore, since the control unit 60 turns on the energization circuit 35, the energization circuit 35 enters the energization state for energizing the base material 34a, and the temperature of the conductive polymer film 34b increases, thereby promoting the release of the fine water. The released fine water is supplied into the supply tank 50 together with air and is radiated on the base B. As described above, since the base B is irradiated with the fine water while being stirred, the fine water can be uniformly supplied to the base B and mixed.

After the control unit 60 performs the moisture release control in S230, the control unit 60 waits for a predetermined moisture release time Tb to elapse (S240), and if the control unit 60 determines that the moisture release time Tb has elapsed, the control unit 60 stops the operation of the stirring device 55 to end the stirring operation of the base B (S250) and determines if the supply of fine water has been completed (S260). If the control unit 60 determines that the supply of fine water is not completed, the control unit 60 returns to S200 to perform the moisture absorption control. In this manner, the control unit 60 alternately repeats the moisture absorption control and the moisture release control. The moisture absorption time Ta and the moisture release time Tb can be appropriately set in accordance with a moisture absorption capacity (moisture release capacity) of the fine water generation cartridge 30, a size of the supply tank 50, a type and amount of the base B, the active ingredient to be contained, and the like. Although not particularly limited, for example, the moisture absorption time Ta is set to about twice the moisture release time Tb, and when the moisture release time Tb is set to 30 seconds or 1 minute, the moisture absorption time Ta is set to 1 minute, 2 minutes, or the like.

Furthermore, in S260, for example, when a process time from the start of the fine water supply process reaches a predetermined time such as several tens of minutes, it may be determined that the supply is completed, or when the number of repetitions of the moisture absorption control and the moisture release control reaches a predetermined number of times, it may be determined that the supply is completed. If the control unit 60 determines in S260 that the supply of the fine water has been completed, the control unit 60 ends the fine water supply process.

In the external preparation producing method in FIG. 3, after the fine water supply process in S110 is performed, the worker takes out, from the supply tank 50, the external preparation P produced by the fine water being supplied to the base B (S120) and stores the external preparation P in a predetermined container C for the external preparation P (S130). The external preparation producing method is ended. Note that the fine water is mainly contained in the base B, but the fine water may be contained in the active ingredient in the base B or may be contained in both the base B and the active ingredient. A predetermined amount of the thus produced external preparation P is taken out from the container C by a user and applied to skin or hair. Alternatively, the external preparation P may be applied after fine water is supplied to the external preparation P until immediately before use (application).

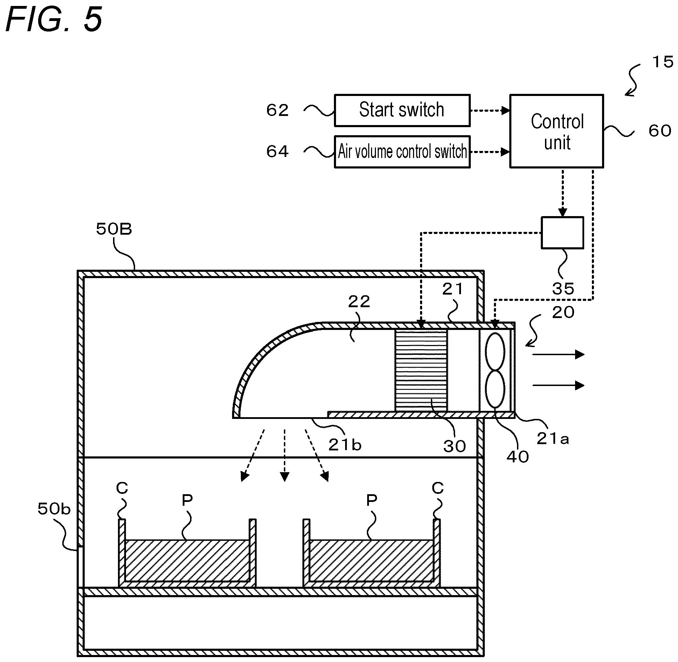

FIG. 5 is a configuration diagram schematically illustrating a configuration of a fine water permeation chamber 15. Similarly to the fine water supply device 10, the fine water permeation chamber 15 includes: a fine water supply unit 20; a supply tank 50B; and a control unit 60, and is disposed at a delivery agent (sales floor) of the external preparation P, an aesthetic salon, and the like. The supply tank 50B does not include a stirring device 55, and containers C from which lids are removed are disposed. Note that the supply tank 50B is configured to be vertically dividable, for example, and the containers C are put in and taken out in a state where the upper sides (lids) are removed. Of course, it may be taken in and out through an opening 50a in a side wall, like the supply tank 50.

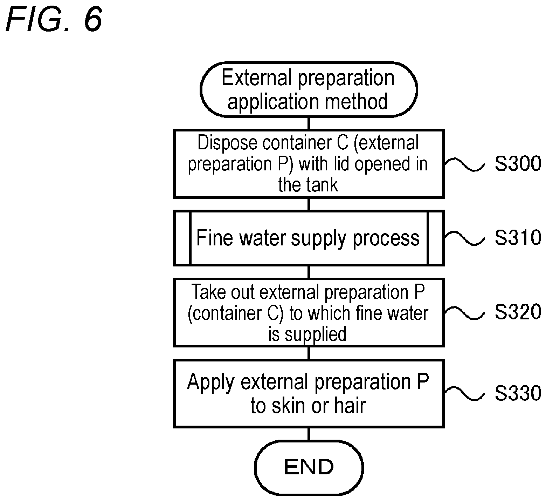

FIG. 6 is a process chart illustrating an example of an external preparation application method. A practitioner (store clerk) of a delivery agent, an aesthetic salon, or the like places the container C (external preparation P) with the lid opened, in the supply tank 50B (step S300). Next, the practitioner operates a start switch 62 of the fine water permeation chamber 15 to perform a fine water supply process by the fine water supply unit 20 (step S310). Step S310 is performed similarly to the fine water supply process in FIG. 4 except that there is no stirring operation, and the description thereof is therefore omitted.

Then, when the fine water supply process is performed for a predetermined time such as, ten and several minutes or several tens of minutes, the practitioner takes out the container C in which fine water is supplied (replenished) to the external preparation P (step S320) and applies the external preparation P to the skin or hair of a person being treated to cause the external preparation P to permeate into the skin or hair by a permeation route formed by the fine water and by a permeation force of the fine water (step S330), and the process of external preparation application is ended. Note that the fine water permeation chamber 15 may be disposed at the home of a purchaser of the external preparation P and at other places, and the purchaser (user) of the external preparation P may perform the external preparation application method of FIG. 6.

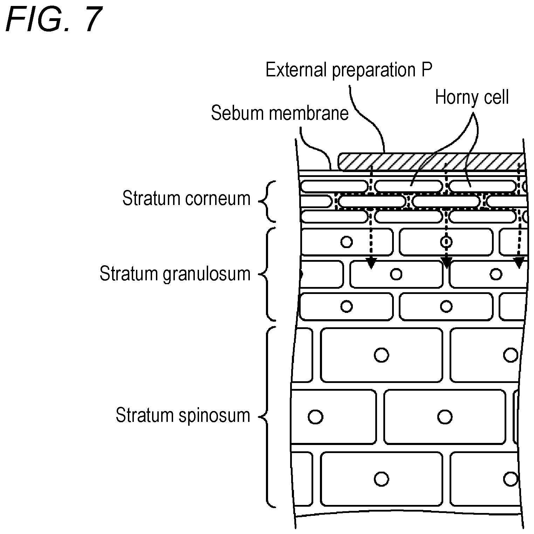

Here, FIG. 7 is an explanatory view illustrating an image in which the fine water permeates into skin. As illustrated, an epidermis of skin includes a stratum corneum covered with a sebum membrane, a stratum granulosum, a stratum spinosum, a basal layer (not illustrated), and the like. When the external preparation P is applied to the skin, the fine water enters the stratum corneum and enters the intercellular lipids between the horny cells (see the arrows in FIG. 7). The horny surface has gaps of about 20 to 50 nanometers or less. Since the fine water has a particle size of 50 nanometers or less and about 1 to 2 nanometers, the fine water easily enters into the stratum corneum. Furthermore, because the skin is generally positively charged, if the water particles are negatively charged, the water particles are electrically attracted by the skin surface and hardly enter into the stratum corneum, and if the water particles are positively charged, the water particles are repelled and hardly reach the skin surface. Because the fine water is not charged, such situations do not occur. The fine water that has thus entered into the stratum corneum forms routes of water along the intercellular lipids around the horny cells. At that time, the moisture amount of the intercellular lipids is improved, and the moisture also enters into the natural moisturizing component in the horny cells; therefore, the moisture amount of the entire stratum corneum can be brought close to an appropriate value, thereby improving the moisture retaining property. In addition, the active ingredient of the external preparation P passes through the routes of water formed in the intercellular lipids by the fine water and diffuses in the skin; therefore, permeation of the active ingredient can be promoted. Note that, in addition to the above-described pattern in which the routes of water are formed of fine water and the medicinal agent is diffused through the routes, there can be considered a pattern of permeation of medicinal agent, in which pattern the medicinal agent is pushed in together with the fine water by the permeation force of the fine water at the same time as the fine water permeates. As described above, the fine water provides the effect of improving the moisture retaining property due to the supply of moisture, and provides synergistically provides the effect of improving the permeability of the active ingredient accompanying the formation of the routes of water formed of fine water or provides the effect of improving the permeability of the active ingredient due to the permeation force of the fine water. How the fine water permeates into the skin is described above. However, the following pattern of permeation of medicinal agent can also be considered. The fine water similarly permeates into and remains in the fine gaps (about 50 nm or less) between cuticles on the hair surface to moisturize the hair, and water routes are formed of fine water. The medical substance diffuses through the water routes, and, in addition, the medicinal agent is pushed in together with the fine water by the permeation force of the fine water.

The external preparation P described above contains fine water having a particle size of less than or equal to 50 nanometers in the base B (or the active ingredient). Therefore, when the external preparation P is applied to skin or hair, the fine water permeates, so that routes of water in which the active ingredient permeates can be formed and so that moisturization can be sufficiently performed. Therefore, it is possible to enhance the moisturizing effect and the permeation effect. Note that the base B may be either fat-soluble or water-soluble, but if the base B is fat-soluble, the base B more easily permeates into skin or the like than the water-soluble base B; therefore, it is possible to further improve the permeability of the active ingredient due the contained fine water.

Furthermore, since the fine water supply device 10 supplies the fine water in the state where the base B is disposed in the supply tank 50, the external preparation P containing the fine water in the base B can be appropriately produced. In addition, since the fine water supply device 10 stirs the base B with the stirring device 55 when the fine water generation cartridge 30 is in the moisture release state, the fine water can be uniformly contained in the base B.

Furthermore, in the external preparation application method, since the fine water is supplied to the external preparation P disposed in the supply tank 50B of the fine water permeation chamber 15 until immediately before application (use), the external preparation P can be applied in the state of sufficiently containing the fine water. Therefore, for example, even when part of the contained fine water evaporates during a period from when the external preparation P is produced to when the external preparation P is used, the fine water can be replenished before application. Therefore, the moisturizing effect and the permeation effect by the fine water can be appropriately exhibited.

### Second embodiment

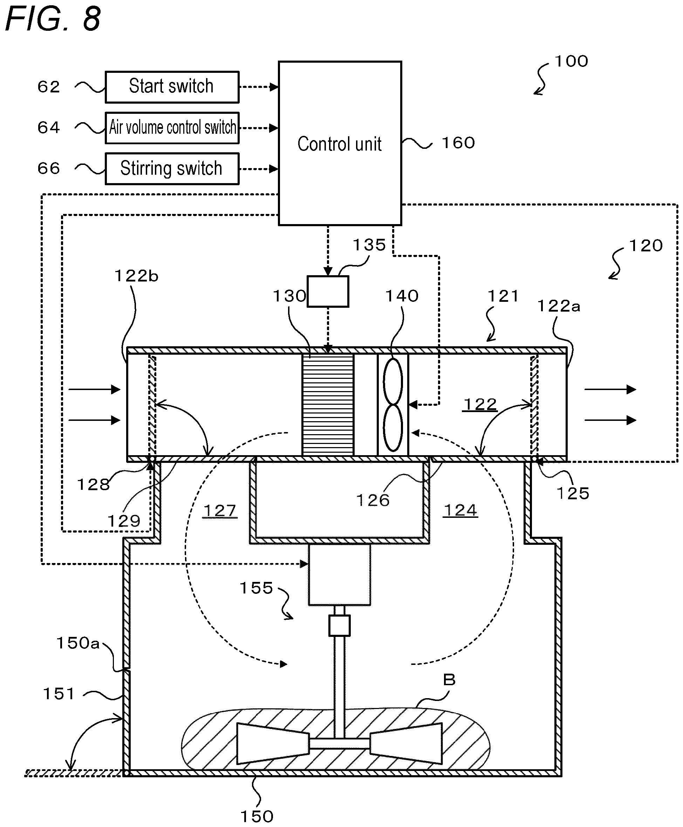

Next, a second embodiment of the present disclosure will be described. FIG. 8 is a configuration diagram schematically illustrating a configuration of a fine water supply device 100 of the second embodiment. The fine water supply device 100 includes a fine water supply unit 120, a supply tank 150, and a control unit 160. The fine water supply unit 120 includes an air passage 121, a fine water generation cartridge 130, an energization circuit 135, and a fan 140, and has the same configuration as the first embodiment except the air passage 121; therefore, the description thereof is omitted.

The air passage 121 includes a main passage 122, a first communication passage 124, and a second communication passage 127, and is provided with a first switching part 125 and a second switching part 128. The main passage 122 is a tubular passage whose both ends are opened. In the main passage 122, the fan 140 and the fine water generation cartridge 130 are provided in this order from a first opening 122a on one side toward a second opening 122b on the other side. The first communication passage 124 and the second communication passage 127 communicate the main passage 122 with the supply tank 150. The first communication passage 124 is connected to the main passage 122 on the first opening 122a side with respect to the fan 140 and extends into the supply tank 150. The second communication passage 127 is connected to the main passage 122 on the second opening 122b side with respect to the fine water generation cartridge 130 and extends into the supply tank 150.

The first switching part 125 includes a switching plate 126 that operates by being driven by a motor (not illustrated), and the second switching part 128 includes a switching plate 129 that operates by being driven by a motor (not illustrated). As indicated by the solid lines in FIG. 8, when the switching plate 126 is at a normal position (initial position), the first switching part 125 closes (blocks) the first communication passage 124 and opens the first opening 122a side to allow air to flow through the first opening 122a. Further, when the switching plate 129 is at a normal position, the second switching part 128 closes (blocks) the second communication passage 127 and opens the second opening 122b side to allow air to flow through the second opening 122b. In this state, the mixing tank 112 is blocked from the main passage 122 and sealed; therefore, this state is referred to as a sealed state (blocked state).

On the other hand, as indicated by the dotted lines in FIG. 8, when driving of the motor causes the switching plate 126 to operate and move to an operation position where the switching plate 126 is rotated by 90 degrees, the first switching part 125 opens the first communication passage 124 and closes the first opening 122a side to block the flow of air through the first opening 122a. Furthermore, when driving of the motor causes the second switching plate 129 to operate and move to an operation position where the switching plate 129 is rotated by 90 degrees, the second switching part 128 opens the second communication passage 127 and closes the second opening 122b side to block the flow of air through the second opening 122b. In this state, the mixing tank 112 and the main passage 122 communicate with each other through the first communication passage 124 and the second communication passage 127, so that air can circulate through the mixing tank 112 and the main passage 122; therefore, this state is referred to as a circulation state (communication state).

Note that the fan 140 rotates in a first rotation direction to blow air from the first opening 122a side toward the second opening 122b side. Furthermore, the fan 140 rotates in a second rotation direction opposite to the first rotation direction to blow air from the second opening 122b side toward the first opening 122a side.

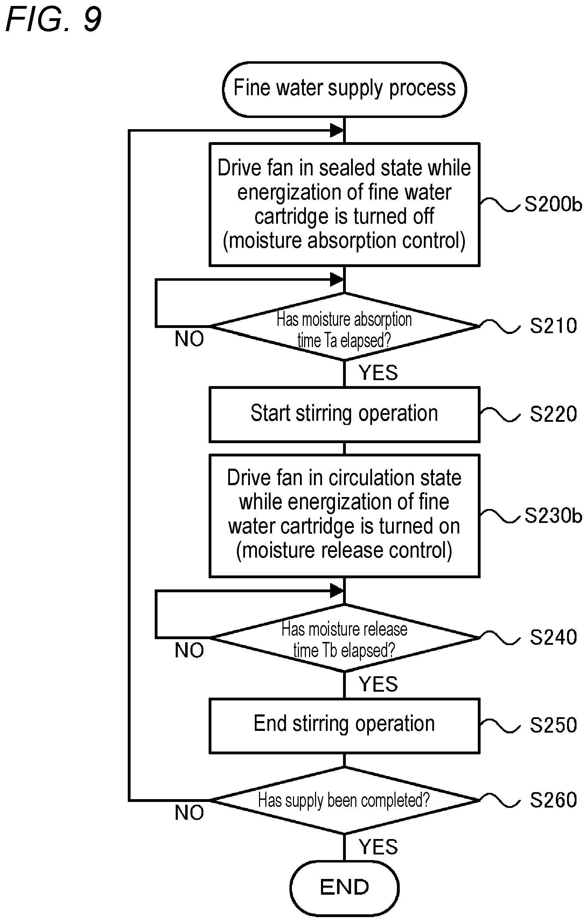

An operation of the fine water supply device 100 configured as described above will be described. Here, the operation in a case of performing the fine water supply process of S 110 of the external preparation producing method in FIG. 3 will be described. FIG. 9 is a flowchart illustrating an example of a fine water supply process according to the second embodiment. In this fine water supply process, the same processing as that in the first embodiment (FIG. 4) is denoted by the same step number, and a detailed description thereof is omitted.

In the fine water supply process, the control unit 160 establishes the above-described sealed state and performs moisture absorption control in which the fan 140 is rotationally driven in the second rotation direction while energization to the fine water generation cartridge 130 is turned off, thereby causing moisture to adsorb to the conductive polymer film 34b (S200b). Therefore, the air sucked into the main passage 122 from the second opening 122b flows toward the first opening 122a (see the solid arrows in FIG. 8), and the temperature of the conductive polymer film 34b decreases since the energization circuit 35 is turned off, so that the adsorption of moisture is promoted. Note that the rotation direction of the fan 140 may be the first rotation direction.

Furthermore, when a stirring operation of a stirring device 155 has been started in S220, the control unit 160 establishes the above-described circulation state and performs moisture release control in which the fan 140 is rotationally driven in the first rotation direction while energization to the fine water generation cartridge 130 is turned on so that the fine water released from the conductive polymer film 34b is supplied into the supply tank 150 (S230b). Therefore, the air sent from the fan 140 circulates such that the air sent from the fan 140 flows from the second communication passage 127 into the supply tank 150 through the fine water generation cartridge 130 and then returns to the main passage 122 through the first communication passage 124 (see the dotted arrows in FIG. 8). As a result, since the fine water is supplied from the fine water generation cartridge 130 into the mixing tank 112 by using the circulating air without causing new air to flow in, the number of particles of the fine water in the mixing tank 112 increases, so that a high concentration environment of the fine water can be established. This makes it possible to cause the base B to sufficiently absorb the fine water.

The external preparation application method using the fine water supply device 100 is performed as follows. First, a practitioner disposes the base B as the target agent in the supply tank 150 (step S100). Next, the above-described moisture absorption control (S200b) and the moisture release control (S230b) are repeated, and the fine water is supplied to the base B in a high concentration environment of fine water. Then, after the fine water is supplied to the base B for a predetermined time (S260), the practitioner takes out the target agent from the supply tank 150 and applies, as the external preparation P, the target agent to the skin or hair of a person to be treated. As described above, in the external preparation application method (second cosmetic treatment method) of the second embodiment, the concentration of the fine water in the supply tank 150 can be increased so that the target agent can sufficiently contain the fine water; therefore, it is possible to further improve the moisturizing effect and the permeation effect when the external preparation P is applied to the skin or hair.

In each of the above-described embodiments, the stirring operation is performed during the moisture release control of the fine water in the fine water supply process, but the present invention is not limited thereto, and the stirring operation may be performed during the moisture absorption control, or the stirring operation may be performed during both the moisture release control and the moisture absorption control. Alternatively, the present invention does not need to include the stirring device 55 or 155, and it is not necessary to include the stirring device 55 or 155. Furthermore, in the process of producing the external preparation P, the external preparation P may be produced by storing the base B in the container C, and the fine water may be supplied by the fine water supply device 10 or 100 in a state where the lid of the container C is opened.

In each embodiment, in the fine water supply process, the fine water is continuously suppled (constantly supplied) while the moisture absorption control and the moisture release control are alternately performed, but the present invention is not limited thereto. For example, an operation time and a non-operation time may be settable so that the fine water may be intermittently supplied by stopping the moisture absorption control and the moisture release control in the non-operation time.

The fine water supply unit 20 or 120 of each embodiment may include a temperature control cartridge (heat exchanger) in addition to the fine water generation cartridge 30 or 130 and the fan 40 or 140. The temperature control cartridge is formed of a metal material in, for example, a corrugated plate shape, a honeycomb shape, a spiral shape, or the like so that the temperature control cartridge has a large heat capacity and high heat exchange efficiency. Then, the air containing the fine water released from the fine water generation cartridge 30 or 130 during the moisture release control may be supplied into the supply tank 50 (50B) or 150 after passing through the temperature control cartridge. In this way, because the air whose temperature has increased in the fine water generation cartridge 30 or 130 is cooled to near room temperature when passing through the temperature control cartridge, the fine water can be appropriately supplied without raising the temperature of the target agent such as the base B.

Each embodiment may have the following configuration. A humidity sensor is provided in the supply tank 50 (50B) or 150, and the humidity detected by the humidity sensor is used as an alternative characteristic of the number of fine water particles, thereby adjusting the moisture absorption time Ta, the moisture release time Tb, and a non-operation time between the moisture absorption control and the moisture release control, adjusting the air volume for the moisture release control and the moisture release control, and determining completion of the supply of the fine water.

The correspondence relationship between the main elements of the embodiments and the main elements of the present disclosure described in the section of SOLUTIONS TO PROBLEMS will be described. In the embodiments, the external preparation application method (S330) corresponds to the first cosmetic treatment method. Furthermore, the fine water supply device 10 (100) corresponds to the "fine water supply device", the fine water generation cartridge 30 (130) corresponds to the "fine water generator ", and the control unit 60 (160) corresponds to the "control unit". The stirring device 55 (155) corresponds to the "stirring unit".

Note that the correspondence relationship between the main elements of the embodiments and the main elements of the present disclosure described in the section of SOLUTIONS TO PROBLEMS is an example for specifically describing forms for carrying out the present disclosure described in the section of SOLUTIONS TO PROBLEMS; therefore, the correspondence relationship does not limit the elements of the present disclosure described in the section of SOLUTIONS TO PROBLEMS. That is, the interpretation of the present disclosure described in the section of SOLUTIONS TO PROBLEMS should be made based on the description in the section, and the embodiments are merely a specific example of the present disclosure described in the section of SOLUTIONS TO PROBLEMS Although the embodiments for carrying out the present disclosure have been described above with reference to the embodiments, the present disclosure is not limited to such embodiments at all, and can be carried out in various forms without departing from the gist of the present disclosure.

### Industrial applicability

The present disclosure can be used for cosmetic treatment in which an external preparation for skin or hair is applied.

## Claims

1. An external preparation for skin or hair comprising:
a predetermined active ingredient; and a base,
wherein at least one of the active ingredient and the base contains fine water that is uncharged and has a particle size of less than or equal to 50 nanometers.

2. A cosmetic treatment method comprising
applying, to skin or hair, an external preparation including a predetermined active ingredient and a base, wherein at least one of the active ingredient and the base contains fine water that is uncharged and has a particle size of less than or equal to 50 nanometers.

3. A cosmetic treatment method comprising:
disposing, in a tank, a base containing a predetermined active ingredient or an external preparation having the base, as a target agent;
increasing a concentration of the fine water in the tank, by repeating a moisture absorption state to adsorb moisture in the air to the conductive polymer film in which air outside the tank is supplied to a conductive polymer film at a lowered temperature while an inside of the tank is sealed, and a moisture release state to cause the moisture adsorbed to the conductive polymer film to be released into the tank as fine water having a particle size of less than or equal to 50 nanometers in which the air in the tank is circulated to the conductive polymer film at a raised temperature;
supplying the fine water to the target agent for a predetermined time; and
taking out the target agent from the tank and applying the target agent to skin or hair as an external preparation.

4. A fine water supply device comprising:
a fine water generator that changes to a moisture absorption state in which moisture in air is made to be adsorbed to a conductive polymer film due to a decrease in temperature and to a moisture release state in which the moisture adsorbed to the conductive polymer film is made to be released into a tank as fine water having a particle size of less than or equal to 50 nanometers due to an increase in temperature; and
a control unit that controls the fine water generator such that, in a state where a base containing a predetermined active ingredient or an external preparation having the base is disposed in the tank as a target agent, the fine water is supplied to the target agent.

5. The fine water supply device according to claim 4, further comprising a stirring unit that stirs the target agent in the tank,
wherein the control unit controls the stirring unit such that the stirring unit stirs the target agent in at least one of the moisture absorption state and the moisture release state.

6. The fine water supply device according to claim 5, further comprising a switching part that selects any one of a sealed state in which communication between the fine water generator and the tank is blocked and the air outside the tank is supplied to the fine water generator, and a circulation state in which communication is established between the fine water generator and the tank and air inside the tank is supplied to the fine water generator,
wherein the control unit brings, in the sealed state of the switching part, the fine water generator unit into the moisture absorption state and brings, in the circulation state of the switching part, the fine water generator into the moisture release state such that a concentration of the fine water in the tank is increased.

7. The cosmetic treatment method according to claim 2, further comprising:
adsorbing moisture to a conductive polymer film having a nanochannel;
increasing a temperature of the conductive polymer film to release the moisture adsorbed to the conductive polymer film; and
causing the fine water to be contained in at least one of the active ingredient and the base.
